# EUROPÄISCHE PATENTSCHRIFT

(11) **EP 0 988 106 B1**
(45) Veröffentlichungstag und Bekanntmachung des Hinweises auf die Patenterteilung: **29.08.2001**
(21) Anmeldenummer: 98930687.3
(22) Anmeldetag: 13.05.1998
(51) Int. Cl.: B01J 2/20, A61K 9/16, B29C 47/64

(54) **VERFAHREN ZUR HERSTELLUNG KLEINTEILIGER ZUBEREITUNGEN BIOLOGISCH AKTIVER STOFFE**
METHOD FOR PRODUCING SMALL-PARTICLE PREPARATIONS OF BIOLOGICALLY ACTIVE SUBSTANCES
PROCEDE DE PREPARATION DE COMPOSITIONS A FINES PARTICULES DE SUBSTANCES BIOLOGIQUEMENT ACTIVES

(30) Priorität: 22.05.1997 DE 19721467
(43) Veröffentlichungstag der Anmeldung: 29.03.2000
(73) Patentinhaber: BASF AKTIENGESELLSCHAFT, 67056 Ludwigshafen (DE)
(72) Erfinder: BREITENBACH, Jörg, D-68199 Mannheim (DE); ZETTLER, Hans, Dieter, D-67269 Grünstadt (DE)
(86) Internationale Anmeldenummer: EP9802821
(87) Internationale Veröffentlichungsnummer: WO9852684

(56) Entgegenhaltungen:
- EP-A- 0 578 603
- EP-A- 0 582 300
- EP-A- 0 629 479
- DE-C- 19 522 899

## Beschreibung

Die vorliegende Erfindung betrifft ein Verfahren zur kontinuierlichen Herstellung kleinteiliger Zubereitungen von biologisch aktiven Stoffen, in denen die biologisch aktiven Stoffe in einer Matrix aus thermoplastisch verarbeitbaren Hilfsstoffen homogen verteilt vorliegen, in einer in mehrere Zonen unterteilten Schneckenmaschine. Weiterhin betrifft die Erfindung eine Vorrichtung zur kontinuierlichen Herstellung entsprechender Zubereitungen.

Die Herstellung von wirkstoffhaltigen Pulvern oder anderen kleinteiligen Formen nach herkömmlichen Verfahren ist wegen der Anzahl der Verfahrensschritte und der Staubproblematik an den Schnittstellen der verschiedenen Verfahrensschritte häufig sehr aufwendig und damit wirtschaftlich uninteressant.

Aus der DE-C 33 32 629 ist ein Verfahren zur Herstellung eines Pulvers aus Polymeren bekannt, wobei die Polymeren in einem Doppelschneckenextruder aufgeschmolzen, gekühlt, vorgebrochen und feingemahlen werden. Dieses Verfahren bezieht sich vor allem auf die Pulverisierung von Polyethylen.

Es ist allgemein bekannt, wirkstoffhaltige Zubereitungen nach dem Verfahren der Schmelzextrusion herzustellen.

In der EP-A 686 392 wird die Herstellung von pharmazeutischen Zubereitungen durch Extrusion wirkstoffhaltiger Mischungen mit anschließendem Kaltabschlag des Extrudats und Zerkleinerung zu einem Granulat beschrieben.

Aus der DE-A 195 22 899 ist ein Verfahren zum kontinuierlichen Ersintern eines pharmazeutischen Granulats bekannt, bei dem die Mischung der Komponenten zunächst im Extruder angesintert wird und dann in Richtung auf die offene Extruderstirn gefördert wird. Das entstehende Granulat wird erforderlichenfals noch gesiebt. Dieses Verfahren erfordert jedoch auf jeden Fall den Einsatz lipoider Komponenten und beschreibt keine gezielte Herstellung von zerkleinerten Formulierungen.

Problematisch an solchen Verfahren ist es, dass beim Abkühlen der geschmolzenen Massen aufgrund der häufig sehr unterschiedlichen physikalischen Eigenschaften zum einen eine Entmischung auftreten kann, zum anderen aber auch bei Verwendung oligomerer oder polymerer Substanzen ein Molekulargewichtsabbau stattfinden kann. Auch die Froäukteinheitlichkeit ist häufig noch unbefriedigend.

Aufgabe der vorliegenden Erfindung war es, ein Verfahren zur kontinuierlichen Herstellung von partikulären Zubereitungen biologisch aktiver Stoffe zu finden, das unabhängig von der Zusammensetzung auf einfache Weise zu stabilen homogenen Zubereitungen führt.

Demgemäß wurde ein Verfahren zur Herstellung kleinteiliger Zubereitungen biologisch aktiver Stoffe, in denen der biologisch aktive Stoff in einer Matrix aus thermoplastisch verarbeitbaren Hilfsstoffen homogen verteilt vorliegt, in einem in mehrere Zonen unterteilten Schneckenextruder,gefunden, welches dadurch gekennzeichnet ist, dass zunächst in einer heizbaren Zone ein Ansintern oder Aufschschmelzen der Matrixhilfsstoffe sowie ein Vermischen der biologisch aktiven Stoffe mit den Matrixhilfsstoffen erfolgt, woran sich in einer Kühlzone das Kühlen, Vorzerkleinern und Feinmahlen der Mischung anschließt, wobei die Schneckengeometrie in der Kühlzone so gewählt ist, dass die Kühlzone als erste Zone eine Förderzone aufweist, woran sich eine Mischzone und/oder eine Knetzone anschließt.

Weiterhin wurde eine Vorrichtung zur Durchführung des erfindungsgemäßen Verfahrens gefunden, welche aus einem Misch- und Kühlaggregat und einem Auffangaggregat besteht, wobei das Misch- und Kühlaggregat und das Auffangaggregat miteinander zu einem nach außen geschlossenen System verbunden sind, und das Misch- und Kühlaggregat aus einem Extruder mit heiz- und kühlbaren Zonen besteht, dessen Austragsöffnung in das Auffangaggregat, welches aus einem mit konischem Auslaufzylinder versehenen zylindrischen Behälter besteht, mündet.

Erfindungsgemäß wird das Verfahren in einem Schneckenextruder durchgeführt. Bei dem Extruder kann es sich um einen Ein- oder Mehrschneckenextruder handeln, bevorzugt um einen Zweischneckenextruder, der besonders bevorzugt gleichsinnig drehend und dichtkämmend ist.

In der Heizzone des Extruders, in der das Mischen und das Aufschmelzen oder Ansintern erfolgt, kann die Schneckengeometrie dichtkämmend, kämmend oder nichtkämmend gewählt werden, wobei eine dichtkämmende Schneckengeometrie bevorzugt ist. Die Schnekken können sich gegenläufig oder bevorzugt gleichsinnig drehen. Im Misch- und Schmelzbereich sind neben Förderelementen auf den Schnecken bevorzugt Misch- und Knetelemente angeordnet. Förderelemente sind ein- und mehrgängige Schneckenelemente unterschiedlicher Steigung. Mischelemente sind zahnradähnliche Zahnscheibenelemente oder mit Durchbrüchen versehene rückwärtsfördernde Elemente, wobei die Durchbrüche teilwise bis zum Schneckenkern reichen können oder zumindest die Hälfte des Helixradius ausmachen. Knetelemente sind Zwei- oder Dreispitzscheiben, wobei die Elemente immer mehrere Scheiben mit unterschiedlicher Breite und unterschiedlichem Versatzwinkel zueinander besitzen.

Die Temperatur in der Misch- und Aufschmelzzone kann je nach den zu verarbeitenden Mischungen im Bereich von 18 bis 300, vorzugsweise 30 bis 200°C liegen.

Die sich an die Heizzone anschließende Kühlzone besteht im wesentlichen zunächst aus einer Förderzone, an die sich eine Mischzone und/oder eine Knetzone anschließen.

Entscheidend für den Verfahrenserfolg ist es, im ersten Teil der Kühlzone reine Förderelemente einzusetzen, um einen möglichst geringen Energieeintrag und eine geringere Scherbelastung zu erzielen und eine möglichst rasche Abkühlung der Schmelze unter den Erweichungspunkt zu erreichen. An den Förderbereich der Kühlzone kann sich direkt eine Knetzone zur Zerkleinerung der Masse anschliessen, vorzugsweise jedoch in Fliessrichtung zunächst eine Mischzone mit Mischelementen und darauf folgend eine Knetzone zur Zerkleinerung der Masse.

Der Mantel der Kühlzone wird mit einem flüssigen Kühlmedium gekühlt. In der Förderzone der Kühlzone wird die Temperatur bevorzugt auf 5 bis 30°C unter die Erweichungstemperatur der zu kühlenden Masse eingestellt. Über die gesamte Kühlzone kann die Temperatur in Fliessrichtung je nach Erweichungspunkt der Massen um bis zu 150°C unter den Erweichungspunkt abgesenkt werden. Es kann sich auch empfehlen, im Mischbereich der Kühlzone eine Schockkühlung vorzunehmen und den Mantel auf Temperaturen im Bereich von -10°C bis +10°C zu kühlen.

Zur Vermeidung von starken Temperaturgradienten über den Gangquerschnitt sind besonders bevorzugt nach einem Drittel der Kühlzonenlänge bevorzugt Mischelemente einzusetzen, beispielsweise gegensinnig fördernde Elemente mit breiten Durchbrüchen, die eine Masseumschichtung bewirken. Nach Absenkung der Massetemperatur unter den Erweichungspunkt wird im letzen Drittel der Kühlzone durch Einsatz von Zwei- oder Dreispitzknetscheiben, die gegebenenfalls durch Förderelemente unterbrochen sein können, die Zerkleinerung und Vermahlung-der erstarrten Masse zu partikulären Zubereitungen vorgenommen.

Zwischen Mischzone und Knetzone können gegebenenfalls auch noch kurze Förderelemente eingebaut werden, ebenso wie es sich empfehlen kann, zwischen den Mischelementen oder den Knetelementen kurze Förderstrecken vorzusehen.

Die detaillierte Schneckengeometrie richtet sich auch nach der Reihenfolge der Zugabe der Komponenten sowie in speziellen Fällen nach der Art der verwendeten Hilfsmittel.

Im Falle, dass eine Vormischung aus Matrixmaterialien, Additiven und biologisch aktiven Substanzen in den Extruder eingebracht wird, wird die Schneckengeometrie der Misch- und Aufschmelzzone (Heizzone) vorzugsweise so gewählt, dass zunächst Förderelemente die Mischung weiterfördern, dann ein Aufschmelzen der Mischung in einem Bereich erfolgt, in dem vorwiegend Knetelemente, gegebenenfalls auch Rückförderelemente, vorliegen, woran sich in der nun folgenden Kühlzone zunächst eine Förderzone, eine Mischzone und eine Zerkleinerungszone anschließen.

In einer anderen möglichen Verfahrensgestaltung werden zunächst Matrixhilfsstoffe und weiter Additive in den Extruder dosiert, mit Hilfe von Förderelemnten in Fließrichtung gefördert und in einem vorwiegend durch Mischelemente geprägten Bereich aufgeschmolzen. Danach wird eine Mischung aus biologisch aktiver Substanz und gegebenfalls einem Trennmittel zudosiert und in einem weiteren Mischbereich mit der Schmelze homogenisiert. In dem Homogenisierungsbereich kann die Temperatur grösser oder kleiner als im ersten Mischbereich sein. Bevorzugt ist sie niedriger. Die homogene Schmelzemischung wird sodann in der Kühlzone abgekühlt und zerkleinert. Durch die Zugabe eines Trennmittels können Fehlstellen im erkaltenden Material entstehen, die den Zerkleinerungsvorgang erleichtern.

Eine weitere Verfahrensgestaltung bezieht sich auf Mischungen, denen ein Treibmittel zudosiert wird. Eine Vormischung aus Matrixhilfsstoffen und biologisch aktiven Stoffen wird in den Extruder dosiert, in Fließrichtung gefördert und aufgeschmolzen. Innerhalb der Heizzone schließt sich an die Aufschmelzzone eine Förderzone und dann eine Mischzone an, in der die Zugabe des Treibmittels erfolgt. Daran schließt sich die Kühlzone an. Die Mischzone der Heizzone und der erste Teil der Kühlzone (Förderzone) sind in Fließrichtung durch Stauelemente abgesperrt. Stauelemente sind rückwärtsfördernde Elemente oder Knetscheiben mit Rückfördercharakter. Durch den Einsatz der Stauelemente wird ein Druck erzeugt, so dass das Aufschäumen erst in der zweiten Zone der Kühlzone (Mischzone) erfolgt. Der Aufschäumvorgang unterstützt den Zerkleinerungsvorgang wirkungsvoll.

An die Kühlzone schließen sich noch Förderelemente an, um die erkaltete und zerkleinerte Masse aus dem Extruder auszutragen. Die Produkte können über einen offenen Extruderkopf ausgetragen werden. In einer bevorzugten Ausführungsform ragen die Förderelemente am Extruderauslass über den Schneckenkanal hinaus, vorzugsweise um das 0.5 bis 1.5-fache des Schneckendurchmessers. Weiterhin kann auch ein einfacher Zylinderflansch als Übergangsstück den letzten Extruderflansch mit einem weiterführenden Flansch einer Sammelvorrichtung verbinden. Dabei ist es bevorzugt, dass in diesem Übergangsflansch die partikulären Produkte der beiden Schneckenkanäle zusammengeführt werden, so dass eine einzige Bohrung fortan genügt, um den Produktstrom zu leiten. An diesen Übergangsflansch kann auch eine Druckluftvorrichtung angefügt sein, die das Produkt durch einen Luftstrom vom Extruderkopf wegbefördert. Durch den Einsatz eines Luftabscheiders kann später das Produkt von dem Luftstrom getrennt werden.

Mit Hilfe des erfindungsgemäßen Verfahrens lassen sich Zubereitungen mit Korngrössen im Bereich von 0.001 bis 50 mm Durchmesser herstellen. Je nach Wahl der Schneckendurchmesser, der Misch- und Knetelemente und der Schneckendrehzahlen erhält man großkörnige Partikel (10 bis 50 mm), mittelkörnige (1 bis 3 mm), kleinkörnige (0.3 bis 1 mm), feinkörnige (0.1 bis 0.3 mm), dichtkörnige (0.03 bis 0.1 mm) oder mikrokristalline (0.001 bis 0.03 mm) Partikel. Bevorzugt werden Korngrössen von 0.001 bis 10, besonders bevorzugt 0.1 bis 3 mm. Welche Korngrössen man im einzelnen einstellt, richtet sich vor allem nach dem gewünschten Anwendungsbereich. Die partikulären Zubereitungen weisen eine gute Einheitlichkeit in der Korngrössenverteilung auf, so dass sie ohne weitere Siebvorgänge weiterverarbeitet werden können. Dies lässt sich durch Siebanalyse ermitteln. Eine gute Einheitlichkeit der Korngrössenverteilung ist für die Fliesseigenschaften der Produkte vorteilhaft und insbesondere für die Direkttablettierbarkeit der Pulver oder Granulate von Bedeutung.

Entscheidend für die Produktqualicät ist auch die Masseeinheitlichkeit der Zubereitungen, da es nicht nur zu hohe Staubanteile zu vermeiden gilt, sondern vor allem auch eine Entmischung der Komponenten. Dies ist vor allem für die Lagerstabilität der Produkte von Bedeutung. Durch die spezifische Wahl der Schneckengeometrie in der Kühlzone kann eine Entmischung der Komponenten ebenso vermieden werden, wie ein Molekulargewichtsabbau oligomerer oder polymerer Bestandteile der Mischung.

Die Erfindung betrifft auch eine Vorrichtung zu kontinuierlichen Herstellung von Zubereitungen biologisch aktiver Stoffe, welche aus einem Mischaggregat und einem Auffangaggregat besteht, wobei Misch- und Auffangaggregat zu einem nach außen geschlossenen System verbunden sind, und das Mischaggregat aus einem wie oben beschriebenen Schneckenextruder mit Heiz- und Kühlzone zum Vermischen und Zerkleinern der Komponenten besteht, wobei die Austragsöffnung des Mischaggregats in das Auffangaggregat, welches aus einem mit konischem Auslauftrichter versehenen zylindrischen Behälter besteht, mündet. Mischaggregat und Auffangaggregat können durch eine Schweissnaht oder vorzugweise über einen Flansch miteinander verbunden sein.

Durch diese Anordnung wird auf einfache Weise die Kontamination der Zubereitungen durch Verunreinigungen aus der Raumluft verhindert. Dies ist vor allem bei der Herstellung von pharmazeutischen Zubereitungen, die GMP-Anforderungen genügen müssen (GMP: Good Medical Practice) von grossem Vorteil.

In einer bevorzugten Ausgestaltung der Vorrichtung mündet der konische Auslauftrichter des Auffangaggregats direkt in eine Förderschnecke, durch die das partikuläre Material abtransportiert wird. In einer weiteren Ausgestaltung desr Vorrichtung schließt sich an die Förderschnecke unmittelbar eine Verpackungs- oder Formgebungseinreit an.

Als Formgebungseinheit kann eine konventionelle Tablettenpresse oder eine Verkapselungsvorrichtung eingesetzt werden. Weiterhin kann als Formgebungseinheit auch ein weiterer Schneckenextruder mit Kalandriereinrichtung dienen, in der die partikuläre Zubereitung mit weiteren Hilfstoffen und/oder biologisch aktiven Substanzen vermischt und, aufgeschmolzen und in noch thermoplastischen Zustand in der Kalandriereinrichtung geformt wird. Dieses Verfahren ist besonders vorteilhaft, wenn die biologisch aktive Substanz vor der Einarbeitung in die endgültige Form beispielsweise in eine spezifische Matrix eingearbeitet werden soll oder zur Verarbeitung untereinander unverträglicher Stoffe.

Die partikulären Zubereitungen können aber auch in einer Verpakkungseinheit direkt in Tonnen, Kannen, Big Bags, Beutel oder Säcke verpackt werden.

Mit Hilfe der erfindungsgemäßen Vorrichtung lassen sich auf einfache Weise on-line beliebige Zubereitungsformen erhalten.

Selbstverständlich ist es auch möglich, die im Extruder erzeugten partikulären Zubereitungen nicht erst zu isolieren, sondern im Anschluss an den Zerkleinerungsschritt in der Kühlzone diese direkt im Extruder weiterzuverarbeiten. Dies kann vor allem dann von Bedeutung sein, wenn die biologisch aktiven Substanzen vor der Einarbeitung in die endgültige Darreichungsform kleinteilig vorformuliert werden müssen, um Inkompatibilitäten mit der Matrix der Darreichungsform zu vermeiden. So kann sich an die Mischund/oder Knetzone eine weitere Extruderzone anschliessen, in der die Pulver oder Granulate mit weiteren Matrixhilfsstoffen vermischt werden, insbesondere mit niedrigschmelzenden Hilfsstoffen wie Polyethylenglykolen, Fetten oder Wachsen, beispielsweise zur Herstellung von Pflastern, Zäpfchenmatrices oder Gelen. Die so erhaltenen plastischen Massen können dann auf an sich bekannte weise durch eine Düse oder Lochplatte extrudiert werden und durch Heiss- oder Kaltabschlag, Kalandrierung, Folienziehen oder Folienblasen geformt werden.

Das erfindungsgemäße Verfahren und die erfindungsgemäße Vorrichtung eignen sich zur Herstellung von partikulären Zubereitungen biologischer Substanzen. Biologisch aktive Substanzen sind erfindungsgemäß Stoffe, die in lebenden Organismen eine biologische Wirkung hervorrufen.

Das erfindungsgemäße Verfahren eignet sich beispielsweise zur Formulierung folgender Stoffe oder deren physiologisch akzeptablen Salzen, wobei die Salze auch in situ im Extruder erzeugt werden können:
- Antiinfektiva
   Aciclovir, Aminoglykoside, Amphotericin B, Azol-Antimykotika, Clotrimazol, Itraconazol, Sepraconazol, Clindamycin, Cephalosporine, Chloramphenicol, Erythromycin, 5-Fluoruracil, Etoposid, Flucytosin, Ganciclovir, Griseofulvin, Gyrasehemmstoffe, Isoniacid, Lincosamide, Mebendazol, Mefloquin, Metronidazol, Nitroimidazole, Novobiocin, Platinverbindungen, Polymyxin B, Praziquantel, Pyrimethamin, Rifamipicin, Saquinavir, Streptomycin, Sulfonamide, Tetracycline, Trimethoprim, Vancomycin, Zidovudin;
- Antipyretika, Analgetika, antiinflammatorische Mittel, Paracetamol, Ibuprofen, Ketoprofen, Oxaprozin, Acetylsalicylsäure, Morphin, Oxaprozin, Propoxyphen, Phenylbutazon;
- Antibiotika
   Rifampicin, Griseofulvin, Chloramphenicol, Cycloserin, Erythromycin, Penicilline wie Penicillin G, Streptomycin, Tetracyclin;
- Antiepileptika
   Hydantoine, Carbamazepin;
- Antitussiva und Antiasthmatika
   Diphenhydramin;
- Antirheumatika
   Chloroquin, Indomethacin, Goldverbindungen, Phenylbutazon, Oxyphenylbutazon, Penicillinamin;
- Hypnotika
   Barbiturace, Phenobarbital, Zolpidem, Dioxopiperidine, Ureide;
- Insektizide
   Aldrin, Dieldrin, Chlorphenothan, Hexachlorcyclohexan;
- Herbizide
   Vinclozolin, Strobilurine;
- Psychopharmaka, Neuroleptika
   Perazin, Promazin, Sulpirid, Thioridazin, Chlorpromazin, Meprobamat, Triflupromazin, Melperon, Clozapin, Risperidon, Reserpin;
- Tranquillantien;
- Antidepressiva
   Imipramin, Paroxetin, Viloxazin, Moclobemid;
- Psychotonika;
- Psychomimetika;
- Diuretika
   Kaliumcanrenoat, Schleifendiuretika, Furosemid, Hydrochlorothiazid, Spironolacton, Thiazide, Triamteren;
- Hormone
   Androgene, Antiandrogene, Gestagene, Glucocorticoide, Oestrogene, Cortisol, Dexamethason, Prednisolon, Testosteron, Adiuretin, Oxytocin, Somatropin, Insulin;
- Immunsuppresiva
   Ciclosporin;
- Bronchodilatoren;
- Muskelrelaxantien, Tranquillantien
   Carisoprodol, Tetrazepam, Diazepam, Chlordiazepoxid;
- Enzyme
   Lipase, Phytase;
- Gichtmittel
   Allopurinol, Colchicin;
- Antikoagulatien
   Cumarine;
- Antiepileptika
   Phenytoin, Phenobarbital, Primidon, Valproinsäure, Carbamazepin;
- Antihistaminika
   Chlorphenoxamin, Dimenhydrinat;
- Antimimetika;
- Antihyperttonika, Antiarryhythmika
   Lidocain, Procainamid, Chinidin, Calciumanatagonisten, Glyceroltrinitrat, Isosorbiddinitrat, Isosorbid-5-monoitrat, Pentaerythrityltetranitrat, Nifedipine, Diltiazem, Felodipin, Verapamil, Reserpin, Minoxidil, Reserpin, Captopril, Enalapril, Lisinopril;
- Sympathomimetika
   Norfenefrin, Oxedrin, Midodrin, Phenylephrin, Isoprenalin, Salbutamol, Clenbuterol, Ephedrin, Tyramin, Isoprenalin, β-Blocker wie Alprenolol, Metoprolol, Bisoprolol;
- Antidiabetika
   Biguanide, Sulfonylharnstoffe, Carbutamid, Tolbutamid, Glibenclamid, Metformin, Acarbose, Troglitazon;
- Eisenpräparationen;
- Vitamine
   Vitamin C, B, A, D, Folsäure;
- ACE-Hemmer
   Captopril, Ramipril, Enalapril;
- Anabolika;
- Iod-Verbindungen;
- Röntgenkontrastmittel;
- ZNS-aktive Verbindungen;
- Antiparkinsonmittel
   Biperiden, Benzatropin, Amantadin, opioide Analgetika, Barbiturate, Benzodiazepine, Disulfiram, Lithiumsalze, Theophyllin, Valproinat, Neuroleptika;
- Zytostatika;
- Antispasmolytika;
- Vasodilatoren
   Naftidrofuryl, Pentoxifyllin.

Es können auch Zubereitungen der biologisch aktiven Stoffe in Form "fester Lösungen" erhalten werden. Der Begriff "feste Lösungen" ist dem Fachmann geläufig (s. Chiou und Riegelman, J. Pharm. Sci. 60, 1281-1302 (1971)). In festen Lösungen von pharmazeutischen Wirkstoffen in Polymeren oder anderen Matrices liegt der Wirkstoff molekulardispers in der Matrix vor.

Die Wirkstoffgehalte können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkombination im Bereich von 0,1 bis 98, vorzugsweise von 0,5 bis-70 Gew.-% liegen. Gleichfalls gelten diese Angaben auch für den Bereich von Nahrungsergänzungsmitteln wie z.B. Vitaminpräparaten.

Als Hilfsstoffe für die Matrix können die folgenden Substanzen eingesetzt werden:

Prinzipiell sind alle durch Schmelzen erweichbare Substanzen als aufnehmende Matrix einsetzbar. Handelt es sich um Polymere können sie gegebenenfalls auch durch Zusatz geeigneter Hilfsmittel bei niedrigeren Temperaturen thermoplastisch verarbeitbar sein.

Die Matrix, in die Partikel während des Extrusionsprozesses eingebettet werden können, kann z.B. aus Polymeren wie Polyvinylpyrrolidon oder Copolymeren des Vinylpyrrolidon mit Vinylacetat, Acrylsäure oder Acrylsäureestern, z.B. Methylacrylat-Ethylacrylat-Copolymere, Polyethylen, Polyisobutylen. Polyethylenglykolen, Polyethylenoxid, Polyethylenglykolpropylenglykol-Copolymeren, Polyvinylalkohol, Polyvinylacetat, teilverseiftes Polyvinylacetat, Celluloseethern wie z.B. Ethyl-, Methyl- oder Hydroxypropylcelluloseether, Hydroxypropylcellulose (Klucel-Marken der Fa. Hercules), Hydroxypropylmethylcellulose, Hydroxypropylcellulose oder Celluloseestern, Hydroxypropylmethylcellulosephthalat, Gelatine, Alginate und ALginsäuren, Pektine, Chitin, Chitosan, Vinylacetat-Ethylen-Copolymere, Vinylacetat-Crotonsäure-Copolymere oder Mischungen dieser Polymere bestehen. Die Matrixpolymere sind vorzugsweise wasserlöslich, zumindest aber wasserquellbar. "Wasserlöslich" heißt, dass sich in 100 g Wasser von 20°C mindestens 0,5 g, vorzugsweise mindestens 2 g des Polymeren lösen, gegebenenfalls auch kolloidal oder micellar.

Weiterhin sind auch Polymermatrizes denkbar, die im Körper resorbiert oder aber abgebaut werden. Dazu gehören Polymilchsäure und deren Copolymere und z.B. Poly(ortho)ester sowie Polyamide, Polyphosphazene und Polyurethane.

Geeignet sind aber auch die Matrizes, die man aus Zuckeralkoholen wie Erythrit, Sorbit, Mannit, Isomalt, Zucker- bzw. Mono- und Disacchariden wie Fructose und Glucose, oder aber Fettsäureglyceriden und/oder Fettsäurepolyethylenglykolestern, wie sie z.B. unter den Namen Gelucire® (Gattefossé) oder Precirole® vertrieben werden, gewinnt. Insbesondere sind auch Stärken und deren Abbauprodukte wie z.B. Maltodextrine oder natürliche Cellulosen verwendbar.

Pharmahilfsstoffe sind z.B: Füllstoffe, Schmiermittel, Formentrennmittel, Weichmacher,- Treibmittel, Stabilisatoren, Farbstoffe, Streckmittel, Fließmittel sowie deren Mischungen. Grundsätzlich jedoch dürfen diesen Pharmahilfsstoffe nicht den erfindungsgemäßen Gedanken einer sich in den Verdauungssäften mit einer Gelschicht umgebenden, sukzessive auflösenden bzw. wenigstens erodierenden, zerfallenden Arnzeiform einschränken.

Beispiele für Füllstoffe sind anorganische Füllstoffe wie die Oxide von Magnesium, Aluminium, Silizium, Titan etc. in einer Konzentration von 0,02 bis 50, vorzugsweise von 0,20 bis 20 Gew.-% bezogen auf das Gesamtgewicht der Arzneiform.

Beispiele für Schmiermittel sind Stearate von Aluminium, Calcium und Magnesium sowie Talkum und Silicone in einer Konzentration von 0,1 bis 5, vorzugsweise von 0,1 bis 3 Gew.-% bezogen auf das Gesamtgewicht der Form.

Als Zerfallsbeschleuniger können z.B. Natriumcarboxymethylstärke oder Crospovidon eingesetzt werden. Auch Benetzungsmittel wie Natriumlaurylsulfat oder Natriumdocusat sind einsetzbar.

Beispiele für Weichmacher beinhalten niedermolekulare Poly(alkylenoxide), wie z.B. Poly(ethylenglycole), Poly(propylenglycole), Poly(ethylenpropylenglycole); organische Weichmacher mit niederem Molekulargewicht wie Glycerin, Pentaerythrit, Glycerinmonoacetat, Diacetat oder Triacetat, Propylenglycol, Natriumdiethylsulfosuccinat etc., zugefügt in Konzentrationen von 0,5 bis 15, vorzugsweise von 0,5 bis 5 Gew.-% bezogen auf das Gesamtgewicht der Arzneiform.

Beispiele für Farbstoffe sind bekannte Azofarbstoffe, organische und anorganische Pigmente oder Farbmittel natürlicher Herkunft.

Anorganische Pigmente sind bevorzugt in Konzentrationen von 0,001 bis 10, vorzugsweise von 0,5 bis 3 Gew.-% bezogen auf das Gesamtgewicht der Arzneiform enthalten.

Darüberhinaus können noch andere Additive zugefügt werden, die die Fließeigenschaften der Mischung verbessern oder als Formtrennmittel wirken, wie z.B: tierische oder pflanzliche Fette, bevorzugt in ihrer hydrierten Form, besonders solche, die bei Raumtemperatur fest sind. Diese Fette haben vorzugsweise einen Schemlzpunkt von 50°C oder höher. Bevorzugt sind Triglyceride der C₁₂-, C₁₄-, C₁₆- und C₁₈-Fettsäuren. Die gleiche Funktion können auch Wachse wie z.B. Carnaubawachs erfüllen. Diese Additive können alleine ohne Zusatz von Füllstoffen oder Weichmachern zugesetzt werden. Diese Fette und Wachse können vorteilhaft allein oder zusammen mit Mono- und/oder Diglyceriden oder Phosphatiden, besonders Lecithin beigemischt werden. Die Mono- und Diglyceride stammen vorzugsweise von den oben beschriebenen Fett-Typen ab, d.h. C₁₂-, C₁₄-, C₁₆- und C₁₈-Fettsäuren. Die Gesamtmenge an Fetten, Wachsen, Mono- und Diglyceriden und/oder Lecithinen beträgt 0,1 bis 30, vorzugsweise 0,1 bis 50 Gew.-% bezogen auf das Gesamtgewicht der Arzneiform.

Als Fließregulierungsmittel können z.B. Aerosile oder Talkum Verwendung finden.

Ferner können auch Stabilisatoren zugefügt werden, wie z.B. Antioxidantien, Lichtstabilisatoren, Hydroperoxid-Vernichter, Radikalfänger und Stabilisatoren gegen mikrobiellen Befall.

Unter Hilfsstoffen im Sinne der Erfindung sind auch Substanzen zur Herstellung einer festen Lösung mit dem pharmazeutischen Wirkstoff zu verstehen. Diese Hilfsstoffe sind beispielsweise Pentaerythrit und Pentaerythrit-tetraacetat, Polymere wie z.B. Polyethylen- bzw. Polypropylenoxide und deren Blockcopolymere (Poloxamere), Phosphatide wie Lecithin, Homo- und Copolymere des Vinylpyrrolidons, Tenside wie Polyoxyethylen-40-stearat sowie Zitronen- und Bernsteinsäure, Gallensäuren, Sterine und andere, wie z.B. bei J.L. Ford, Pharm. Acta Helv. 61, 89-88 (1986) angegeben.

Als pharmazeutische Hilfsstoffe gelten auch Zusätze von Basen oder Säuren zur Steuerung der Löslichkeit eines Wirkstoffes (s. z.B. K. Thoma et al., Pharm. Ind. 51, 98-101 (19891).

Bei Verwendung von polymeren Bindemitteln sollten diese in der Gesamtmischung aller Komponenten im Bereich von 10 bis 250, vorzugsweise von 30 bis 180°C erweichen oder schmelzen oder sinterbar sein, so dass die Masse extrudierbar ist. Die Schmelzen sind vorzugsweise lösungsmittelfrei.

Vorteilhaft ist auch generell das Zumischen von einer oder mehreren Substanzen, die als Treibmittel fungieren können, so zum Beispiel der Zusatz von Citronensäure oder basischen Substanzen wie Carbonaten, speziell Alkalicarbonaten. Treibmittelwirkung kann auch durch Zusatz von basischen Verbindungen zu sauren Wirkstoffen bzw. Säuren zu basischen Wirkstoffen erzielt werden. Weiterhin können auch gasförmige Treibmittel zu den aufgeschmolzenen Massen zugesetzt werden.

Erfindungsgemäß eignet sich das Verfahren zur Herstellung von partikulären pharmazeutischen Mischungen, kosmetischen Formulierungen, Pflanzenschutzmitteln, Düngemitteln, veterinärmecizinischen Mischungen, Tierernährungsmitteln, beispielsweise Fischfutter, oder Nahrungsergänzungsmitteln sowie diätetischen Nahrungsmitteln.

Die pharmazeutischen Mischungen können beispielweise Puder oder Salbenbestandteile sein, weiterhin Trinkgranulate, Sachets oder Grundlagen für Trinksuspensionen oder Sirupe. Vor allem für die Herstellung von Medikamenten für der Pädiatrie haben pulverförmige oder granuläre Zubereitungen ein große praktische Bedeutung.

Die partikulären Zubereitungen lassen sich auch in alle üblichen Arzneiformen einarbeiten, beispielsweise in Tabletten, Dragees, Suppositorien, transdermale Arzneiformen, inhalataorische Arzneiformen wie beispielsweise pulverförmige Asthmamittel.

Das erfindungsgemäße Verfahren eignet sich besonders zur Einarbeitung von Aromastoffen, beispielsweise matrixverkapselten Terpenen.

### Beispiele

### Beispiel 1

Eine Mischung aus 40 Gew.-% Ibuprofen und 60 Gew.-% Polyvinylpyrrolidon (K30) wurde in einem Zweischneckenextruder (ZSK 30, Werner Pfleiderer) mit einem Durchsatz von 10 kg/h extrudiert. Die einzelnen Schüsse in der Heizzone hatten eine Temperatur von 40, 70, 90, 100, 100°C. Die Temperatur des ersten Schusses in der Kühlzone betrug 70°C. Die Kühlzone bestand aus zwei Schüssen in deren Bereich die Extruderschnecken reine Förderelemente enthielten, wobei der zweite Schuss eine Temperatur von 60°C aufwies, und einer Zerkleinerungszone aus drei Schüssen. In diesem Bereich waren die Schnecken aus Dreispitzscheiben aufgebaut. Die Temperatur in Förderrichtung der einzelnen Schüsse betrug 50°C, 30°C, 20°C. Es entstand ein Granulat mit einer mittleren Teilchengröße von 0,7 mm. Das Granulat enthält den Wirkstoff in molekularer disperser Form.

### Beispiel 2

Eine Mischung aus 60 Gew.-% Ibuprofen und 40 Gew.-% Maltodextrin CPUR 01612 (Cerestar) wurde in einem Zweischneckenextruder (ZSK 30, Werner Pfleiderer) mit einem Durchsatz von 5 kg/h extrudiert. Die einzelnen Schüsse in der Heizzone hatten eine Temperatur von 60, 80, 9θ, 120, 120°C. Die Temperatur des ersten Schusses in der Kühlzone betrug 60°C. Die Konfiguration und der Aufbau sowie die Temperatur der sich anschliessenden Schüsse wurde wie in Beispiel 1 gewählt. Es entstand ein Granulat mit einer mittleren Teilchengröße von 0,4 mm.

### Beispiel 3

Die Komponenten wurden über Differentialwaagen getrennt dem Extruder zugeführt. Dabei wurden 50 Gew.-% Theophyllin und 40 Gew.-% Hydroxypropylmethylcellulose (Klucel® der Firma Hercules, USA) und 10 Gew.-% Polyethylenoxid (mittels Molekulargewicht 6000, Lutrol® E 6000 der Firma BASF AG) in einem Zweischneckenextruder (ZSK 30, Werner Pfleiderer) mit einem Durchsatz von 8 kg/h excrudiert. Die einzelnen Schüsse in der Heizzone hatten eine Temperatur von 60, 80, 90, 110, 120°C. Die Temperatur des ersten Schusses in der Kühlzone betrug 90°C.

Die Kühlzone bestand aus zwei Schüssen, in deren Bereich die Extruderschnecken reine Förderelemente enthielten. Der zweite Schuss hatte eine Temperatur von 70°C. Die Zerkleinerungszone bestand aus drei Schüssen. In diesem Bereich waren die Schnecken aus Zweispitzscheiben aufgebaut. Die Temperatur in Förderrichtung der einzelnen Schüsse betrug 60°C, 40°C, 25°C. Es entstand ein Granulat mit einer mittleren Teilchengröße von 0,8 mm.

### Beispiel 4-8

| Wirkstoff 1 | Wirkstoff 2 | Polymer 1 | Polymer 2 | Hilfsstoff(2) | Temp. [°C] Zone 1 | Temp. [°C] Zone 2 |
|---|---|---|---|---|---|---|
| Paracetamol 60 Gew.-% | Coffein 10 Gew.-% | Vinylpyrrolidon-Vinylacetat-Copolymer Kollidon® VA 64 5 Gew.-% | Polyethylenoxid (Lutrol E 1500 der Firma BASF AG) 5 Gew.-% | Isomalt 19 Gew.-% 1 % Erdbeeraroma | 40, 70, 90, 100, 100 | 80, 70, 50, 30, 20 |
| Gallopamil-Hydrochlorid 40 Gew.-% | | Ethylcellulose Typ NF 7 der Fa. Dow, USA, 50 Gew.-% | | 10 Gew.-% Mikrokristalline Cellulose (Avicel® der Fa. FMC, UCS) | 60, 80, 90, 100, 130 | 100, 90, 80, 50, 30 |
| Tramadol-Hydrochlorid, 60 Gew.-% | | Polyvinylpyrrolidon, K Wert 17 (Kollidon K 17 der Fa. BASF AG) 3 Gew.-% | 35 Gew.-% Isomalt | 1 Gew.-% vernetztes Polyvinylpyrrolidon (Crospovidone der Fa. BASF AG), 0,5 Gew.-% Lecithin, 0,5 Gew.-% Natriumlaurylsulfat | 66, 75, 85, 98, 111 | 85, 70, 50. 40, 28 |
| Clotrimazol 60 Gew.% | | Vinylpyrrolidon-Vinylacetat-Copolymer Kollidon VA 64 | | 0,3 Gew.-% Natriumlaurylsulfat, 0,2 Gew.-% Aerosil® 200 C der Fa. Degussa AG) | 50, 70, 90, 100, 100 | 80, 60, 40. 30, 25 |
| Acyclovir-Mononitrat 40 Gew.-% | | Polyacrylat (Eudragit® 30 D, der Fa. Röhm) 3 Gew.-% | Hydroxymethylcellulose 55 Gew.-% | 2 Gew.-% Bentonit A, zudosiert in der Mischzone | 80, 90, 90, 140, 150 | 100, 60, 40, 30, 30 |

### Beispiel 9

Kollidon VA 64 wurde in einem Zweischneckenextruder (ZSK 30, Werner Pfleiderer) mit einem Durchsatz von 5 kg/h extrudiert. Dabei wurde die Konfiguration der Schüsse so gewählt, dass zunächst lediglich ein Fördern stattfand. Danach erfolgte das Aufschmelzen. Die einzelnen Schüsse in der Heizzone hatten eine Temperatur von 60, 80, 90, 120, 130°C. In einer nachgeschalteten Mischzone, deren Schneckenelemente ausschließlich aus Förderelementen bestand, wurde der Wirkstoff Ketoprofen mit 2 kg/h über einen Flansch mit Druckausgleich per Differentialwaage zudosiert. Die Temperatur wured in diesem Bereich bei 130°C gehalten. Die Temperatur der ersten Schusses in der Kühlzone betrug 60°C. Anschließend wurde eine Konfiguration und der Aufbau sowie die Temperatur analog Beispiel 1 gewählt. Es entstand ein Granulat mit einer mittleren Teilchengröße von 0,1 mm. DSC Messungen ergaben, dass es sich um eine molekular disperse Einbettung des Wirkstoffes im erkalteten Pulvermaterial handelt, da der Schmelzpeak des Wirkscoffes nicht mehr auftrat.

## Patentansprüche

1. Verfahren zur kontinuierlichen Herstellung von festen,partikulären Zubereitungen biologisch aktiver Stoffe, in denen die biologisch aktiven Stoffe in einer Matrix aus thermoplastisch verarbeitbaren Hilfsstoffen homogen verteilt vorliegen, in einem in mehrere Zonen unterteilten Schneckenextruder, **dadurch gekennzeichnet,** dass zunächst in einer heizbaren Zone ein Aufschmelzen der Matrixhilfsstoffe sowie ein Vermischen der biologisch aktiven Komponenten mit den Matrixhilfsstoffen erfolgt, woran sich in einer Kühlzone das Kühlen, Vorzerkleinern und Feinmahlen der Mischung anschliesst, wobei die Schneckengeometrie in der Kühlzone so gewählt ist, dass die Kühlzone als erste Zone eine Förderzone aufweist, woran sich eine Mischzone und/oder eine Knetzone anschließt.

2. Verfahren nach Anspruch 1, **dadurch gekennzeichnet**, dass der Mantel der Förderzone der Kühlzone auf eine Temperatur von 5 bis 30°C unter der Erweichungstemperatur der wirkstoffhaltigen Mischung gekühlt wird.

3. Verfahren nach Anspruch 1 oder 2, **dadurch gekennzeichnet**, dass die Kühlzone als erste Zone eine Förderzone aufweist, an die sich in Fliessrichtung zunächst eine Mischzone und dann eine Knetzone anschliessen.

4. Verfahren nach einem der Ansprüche 1 bis 3, **dadurch gekennzeichnet,** dass der Mantel der Mischzone und/oder die Knetzone in der Kühlzone auf Temperaturen im Bereich wie von -10 bis +10°C gekühlt wird.

5. Verfahren nach einem der Ansprüche 1 bis 4, **dadurch gekennzeichnet,** dass in der Heizzone nach Aufschmelzen der Mischungskomponenten ein Treibmittelzusatz erfolgt.

6. Verfahren nach einem der Ansprüche 1 bis 5, **dadurch gekennzeichnet,** dass den Mischungen ein Trennmittel zugesetzt wird.

7. Verfahren nach einem der Ansprüche 1 bis 6, **dadurch gekennzeichnet,** dass sich an die Kühlzone eine Förderzone zum Austrag des Pulvers aus dem Extruder anschliesst, wobei die Förderelemente um das 0.5 bis 1.5-fache des Schneckendurchmessers aus dem Schneckenkanal herausragen.

8. Verfahren nach einem der Ansprüche 1 bis 7, **dadurch gekennzeichnet,** dass man einen gleichsinnig drehenden Zweischneckenextruder einsetzt.

9. Verfahren nach einem der Ansprüche 1 bis 8, **dadurch gekennzeichnet,** dass der Austrag aus dem Extruder in den Innenraum eines mit einem konischen Auslauftrichter versehenen zylindrischen Auffangbehälters erfolgt, wobei Extruderauslass und Auffangbehälter ein nach außen geschlossenes System bilden.

10. Verfahren nach Anspruch 9, **dadurch gekennzeichnet**, dass der konische Auslauftrichter in eine Förderschnecke mündet, die die wirkstoffhaltige Pulverzubereitung kontinuierlich einer Formgebungs- oder Verpackungsvorrichtung zuführt.

11. Vorrichtung zur Durchführung des Verfahrens gemäss Anspruch 1, bestehend aus einem Mischaggregat und einem Auffangaggregat, wobei die Misch- und Auffangaggregat miteinander zu einem nach aussen geschlossenen System verbunden sind, und das Mischaggregat aus einem Extruder mit heizbaren und kühlbaren Zonen besteht, dessen Austragsöffnung in das Auffangaggregat, welches aus einem mit einem konischen Auslauftrichter versehenen zylindrischen Behälter besteht, mündet.

12. Vorrichtung nach Anspruch 11, in der die Förderelemence am Extruderauslass über den Schneckenkanal hinaus in den Innenraum der Auffangzone hineinragen.

13. Vorrichtung nach Anspruch 12, **dadurchgekennzeichnet**, dass die Förderelemente um das 0.5 bis 1.5 fache des Schneckendurchmessers in den Innenraum der Auffangzone hineinragen.

14. Vorrichtung nach einem der Ansprüche 11 bis 13, **dadurch gekennzeichnet,** dass der konische Auslauftrichter in eine Förderschnecke mündet.

15. Vorrichtung nach Anspruch 14, **dadurch gekennzeichnet**, dass sich an die Förderschnecke eine Formgebungs- oder Verpakkungseinheit anschließt.

## Claims

1. A process for the continuous production of solid, particulate preparations of bioactive substances, in which the bioactive substances are homogeneously dispersed in a matrix of thermoplastic auxiliaries, in a screw extruder divided into a plurality of zones, wherein there is firstly melting of the matrix auxiliaries and mixing of the bioactive components with the matrix auxiliaries in a heatable zone, after which the mixture is cooled, precomminuted and finely ground in a cooling zone, the screw geometry in the cooling zone being selected so that the cooling zone has a conveying zone as first zone, followed by a mixing zone and/or a kneading zone.

2. A process as claimed in claim 1, wherein the jacket of the conveying zone of the cooling zone is cooled to a temperature which is 5 to 30°C below the softening temperature of the mixture containing active substances.

3. A process as claimed in claim 1 or 2, wherein the cooling zone has a conveying zone as first zone, followed in the direction of flow by first a mixing zone and then a kneading zone.

4. A process as claimed in any of claims 1 to 3, wherein the jacket of the mixing zone and/or the kneading zone in the cooling zone is cooled to temperatures in the range from -10 to +10°C.

5. A process as claimed in any of claims 1 to 4, wherein a blowing agent is added in the heating zone after the components of the mixture have melted.

6. A process as claimed in any of claims 1 to 5, wherein a release agent is added to the mixtures.

7. A process as claimed in any of claims 1 to 6, wherein the cooling zone is followed by a conveying zone to discharge the powder from the extruder, where the conveying elements project out of the screw channel by 0.5 to 1.5 times the screw diameter.

8. A process as claimed in any of claims 1 to 7, wherein a corotating twin-screw extruder is employed.

9. A process as claimed in any of claims 1 to 8, wherein the discharge from the extruder takes place into the interior of a cylindrical collecting container provided with a conical discharge funnel, where the extruder outlet and the collecting container form a system which is closed to the outside.

10. A process as claimed in claim 9, wherein the conical discharge funnel feeds into a conveying screw which delivers the powder preparation containing active substances continuously to a shaping or packaging arrangement.

11. An arrangement for carrying out the process as claimed in claim 1, consisting of a mixing unit and of a collecting unit, where the mixing and collecting units are connected together to form a system which is closed to the outside, and the mixing unit consists of an extruder which has heatable and coolable zones and whose discharge opening feeds into the collecting unit which consists of a cylindrical container provided with a conical discharge funnel.

12. An arrangement as claimed in claim 11, in which the conveying elements at the extruder outlet project beyond the screw channel into the interior of the collecting zone.

13. An arrangement as claimed in claim 12, wherein the conveying elements project into the interior of the collecting zone by 0.5 to 1.5 times the screw diameter.

14. An arrangement as claimed in any of claims 11 to 13, wherein the conical discharge funnel feeds into a conveying screw.

15. An arrangement as claimed in claim 14, wherein the conveying screw is followed by a shaping or packaging unit.

## Revendications

1. Procédé pour la préparation continue de compositions solides en particules de substances possédant une activité biologique dans lesquelles les substances possédant l'activité biologique sont réparties de manière homogène dans une gangue de produits auxiliaires aptes au travail thermoplastique dans une extrudeuse à vis compartimentée en plusieurs zones, caractérisé par le fait que, dans une zone chauffable, il y a d'abord fusion des produits auxiliaires de gangue et mélange du composant possédant l'activité biologique avec les produits auxiliaires de gangue, il y a ensuite, dans une zone de refroidissement, refroidissement, fragmentation préalable et broyage fin du mélange, la géométrie des vis dans la zone de refroidissement étant choisie en sorte que cette zone de refroidissement comporte, en première zone, une zone de transport, qui est suivie d'une zone de mélange et/ou d'une zone de malaxage.

2. Procédé selon la revendication 1, caractérisé par le fait que l'enveloppe de la zone de transport de la zone de refroidissement est refroidie à une température située de 5 à 30°C au-dessous de la température de ramollissement du mélange contenant la substance active.

3. Procédé selon la revendication 1 ou 2, caractérisé par le fait que la zone de refroidissement comporte, en première zone, une zone de transport, laquelle est suivie, en direction de l'écoulement, d'abord par une zone de mélange puis par une zone de malaxage.

4. Procédé selon l'une des revendications 1 à 3, caractérisé par le fait que l'enveloppe de la zone de mélange et/ou la zone de malaxage dans la zone de refroidissement est refroidie à des températures dans l'intervalle de -10 à +10°C.

5. Procédé selon une des revendications 1 à 4, caractérisé par le fait que, dans la zone chauffable, après fusion des composants du mélange, on ajoute un agent porogène.

6. Procédé selon l'une des revendications 1 à 5, caractérisé par le fait que l'on ajoute aux mélanges un agent de démoulage.

7. Procédé selon l'une des revendications 1 à 6, caractérisé par le fait que, après la zone de refroidissement, il y a une zone de transport pour évacuation de la poudre hors de l'extrudeuse, les éléments transporteurs faisant saillie du canal des vis en proportion de 0,5 à 1,5 fois le diamètre des vis.

8. Procédé selon l'une des revendications 1 à 7, caractérisé par le fait que l'on utilise une extrudeuse à deux vis tournant dans le même sens.

9. Procédé selon l'une des revendications 1 à 8, caractérisé par le fait que l'évacuation de l'extrudeuse se fait dans la chambre intérieure d'un récipient récepteur cylindrique équipé d'une trémie de vidange conique, l'évacuation de l'extrudeuse et le récipient récepteur formant un système fermé vers l'extérieur.

10. Procédé selon la revendication 9, caractérisé par le fait que la trémie de vidange conique débouche dans une vis transporteuse qui envoie la composition pulvérulente contenant la substance active en continu à un appareillage de façonnage ou d'emballage.

11. Appareillage pour la mise en oeuvre du procédé selon la revendication 1, consistant en un appareil de mélange et un appareil récepteur, l'appareil de mélange et l'appareil récepteur étant combiné entre eux sous forme d'un système fermé vers l'extérieur, et en ce que l'appareil de mélange consiste en une extrudeuse avec zones chauffables et refroidissables dont l'orifice d'évacuation débouche dans l'appareil récepteur, lequel consiste en un récipient cylindrique équipé d'une trémie de vidange conique.

12. Appareillage selon la revendication 11, dans lequel les éléments transporteurs à l'évacuation de l'extrudeuse font saillie du canal des vis vers la chambre intérieure de la zone réceptrice.

13. Appareillage selon la revendication 12, caractérisé par le fait que les éléments transporteurs font saillie dans la chambre intérieure de la zone réceptrice en proportion de 0,5 à 1,5 fois le diamètre des vis.

14. Appareillage selon l'une des revendications 11 à 13, caractérisé par le fait que la trémie de vidange conique débouche dans une vis transporteuse.

15. Appareillage selon la revendication 14, caractérisé par le fait que la vis transporteuse est suivie d'une unité de façonnage ou d'emballage.
